# EUROPEAN PATENT SPECIFICATION

(11) **EP 1 590 015 B1**
(45) Date of publication and mention of the grant of the patent: **05.01.2011**
(21) Application number: 04702004.5
(22) Date of filing: 14.01.2004
(51) Int. Cl.: A61M 1/16

(54) **An integrated module for extracorporeal blood treatment**
Integriertes Modul für extrakorporale Blutbehandlung
Module intégré pour un traitement sanguin extracorporel

(30) Priority: 07.02.2003 IT MI20030215
(43) Date of publication of application: 02.11.2005
(73) Proprietor: Gambro Lundia AB, 22 643 Lund (SE)
(72) Inventor: DUCHAMP, Jacques, F-69500 Bron (FR); ABERKANE, Aziz, F-69150 Decines (FR); POUCHOULIN, Dominique, F-01390 Tramoyes (FR); MEYSSONNIER, Gabriel, F-38460 Dizimieu (FR); RIBOLZI, Francesco, I-21100 Varese (IT); NERI, Roberto, I-41037 Mirandola (IT)
(74) Representative: Ponzellini, Gianmarco
(86) International application number: PCT/IB2004/000069
(87) International publication number: WO 2004/069310

(56) References cited:
- EP-A- 0 239 255
- EP-A- 0 362 822
- EP-A- 0 611 227
- EP-A- 0 887 100
- EP-A- 1 048 848
- WO-A-98/52629
- US-A- 4 436 620
- US-A- 5 230 614
- US-A- 5 311 908
- US-A- 5 628 731

## Description

### Background of the Invention.

The invention relates to a support element, to an integrated module for extracorporeal treatment of blood comprising the support element, and to an apparatus for extracorporeal treatment of blood equipped with the integrated module. The invention further relates to an assembly process of an integrated module for extracorporeal treatment of blood.

Specifically, though not exclusively, the invention can be usefully applied to the field of extracorporeal treatment of blood for treatment of renal insufficiency.

The prior art teaches apparatus for blood treatment which remove blood from the patient in a line, carry out an extracorporeal treatment on the blood and then return the treated blood to the patient. Apparatus of this type are used for various treatments; for example therapeutic and non-therapeutic plasmapheresis, extracorporeal oxygenation of blood, purification of blood and removal of water in cases of renal insufficiency. The present invention will be described with particular reference to intensive treatment of renal insufficiency, without any limitation being placed on the ambit of the invention to this specific application thereof.

EP 0 611 227 teaches a multifunctional integrated module for application to a multifunction apparatus for intensive treatment of renal insufficiency, in particular for hemodialysis, hemofiltration and hemodiafiltration. The integrated module comprises a support element, a blood treatment device mounted on the support element and a complex of fluid distribution lines cooperating with the treatment device and associated to the support element. The blood treatment device comprises a semi-permeable membrane which separates two chambers. The distribution line complex comprises a blood withdrawal line from the patient (or arterial line) connected to an inlet of a first chamber of the treatment device, a return line (or venous line) of the treated blood to the patient, connected to an outlet of the first chamber, an infeed line of a treatment fluid (for example a dialysis liquid) connected to an inlet of the second chamber of the treatment device, a waste fluid discharge line connected to an outlet of the second chamber, an infusion line of a substitution liquid which is introduced into at least one of the blood lines, an anticoagulant infusion line which is introduced into the arterial line. The support element comprises a plate-shaped body made of press-formed plastic material. The complex of fluid distribution lines is fixed to the support element at gluing points and zones which are predefined on an internal face of the plate-shaped body, while the treatment device is mounted on the external face of the plate-shaped body itself.

During use, the integrated module is mounted on the blood treatment device and set up following a predefined and simple interconnection procedure so that the treatment device is connected, by the distribution lines, to the cardiovascular system of the patient as well as to suitable containers for access and collection to and of the fluids used in the process. Some distribution lines of the module are coupled with respective peristaltic pumps which the apparatus is equipped with. The pumps invoke circulation of the fluids in the lines; the lines are each provided with a U-shaped arched segment, preformed during assembly of the module and intended for coupling to a pump. On mounting the integrated module on the apparatus the various arched segments of the distribution lines are easily couplable about the peristaltic pumps, so that the latter are immediately operative.

The integrated module is of a disposable type, i.e. destined to be disposed of, usually after a first use, and substituted by another.

The above-described integrated module has the advantage of being easily and rapidly installed on the treatment device. The simple and rapid set-up of the module is particularly advantageous for renal insufficiency intensive treatments, in which the personnel at work is often not expert in the use of machines for blood treatment and where the urgent readying and application of the machine is often of vital importance. Similarly, the dismounting of the module is equally rapid and simple.

The prior art as described above is susceptible to improvement at various levels:
- firstly, the fact that the integrated module has to be totally eliminated after use, including parts such as, for example, the plastic support element, which does not come into direct contact with bodily fluids;
- secondly, the assembly of the integrated module, which is a rather delicate stage, as high precision of positioning of the U-segments of the distribution lines on the support element is required, so that correct coupling with the peristaltic pumps can be achieved;
- thirdly, in relation to the long set-up times and high costs of assembly of the integrated module, which must include a relatively complicated and laborious stage of precise positioning and gluing of the various distribution lines in predetermined gluing zones on the support element.

The document EP 239 255 discloses a device according to the preamble of claim 1

### Summary of the Invention.

The present invention provides a support element for an integrated module for extracorporeal blood treatment thanks to which the module itself can be rapidly assembled and mounted on a blood treatment device.

The invention enables a simplification of the operations for assembly of the integrated module, reduces the scope for error in positioning the distribution lines on the support element, improves precision in the couplings between the U-segments of the distribution lines and the peristaltic pumps of the blood treatment apparatus, enables, after use, a simple and practical separation of the support element from the fluid distribution lines, reduces assembly costs and times of the integrated module.

The above objectives are all achieved by a support element made according to one or more of the appended claims.

### Brief Description of the Drawings.

Further characteristics and advantages of the invention will better emerge from the detailed description of a preferred but non-exclusive embodiment of a support element according to the present invention. The description is made herein below with reference to the accompanying figures of the drawings, which are given by way of example and which are non-limiting.

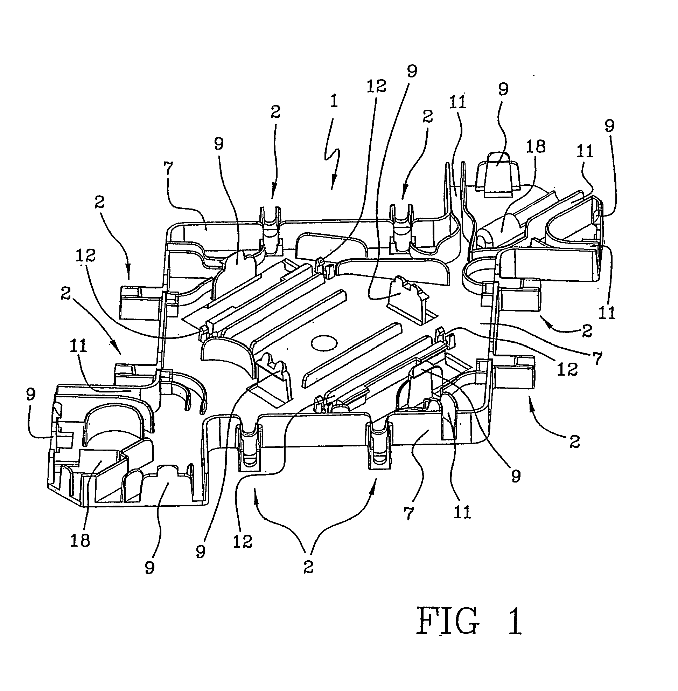

Figure 1 is a perspective view of the internal face of the base body of the support element.

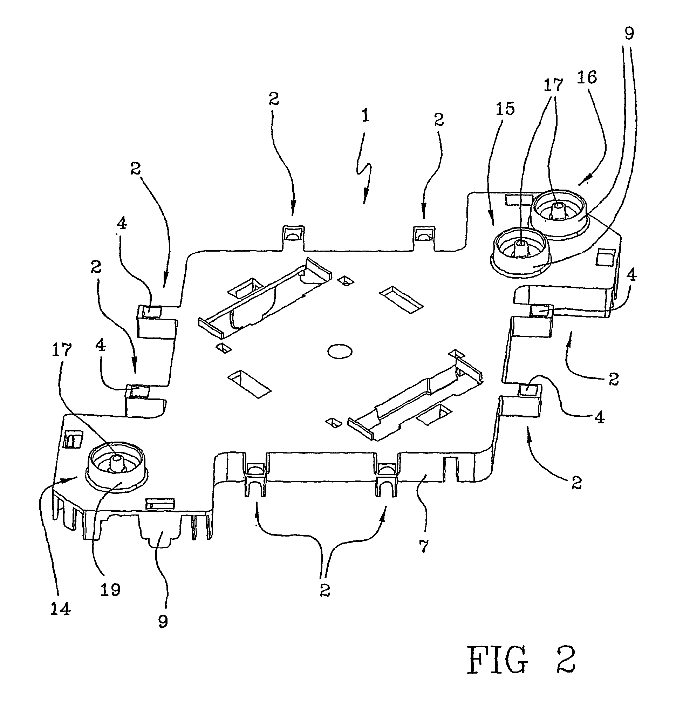

Figure 2 is a perspective view of the external face of the base body of figure 1.

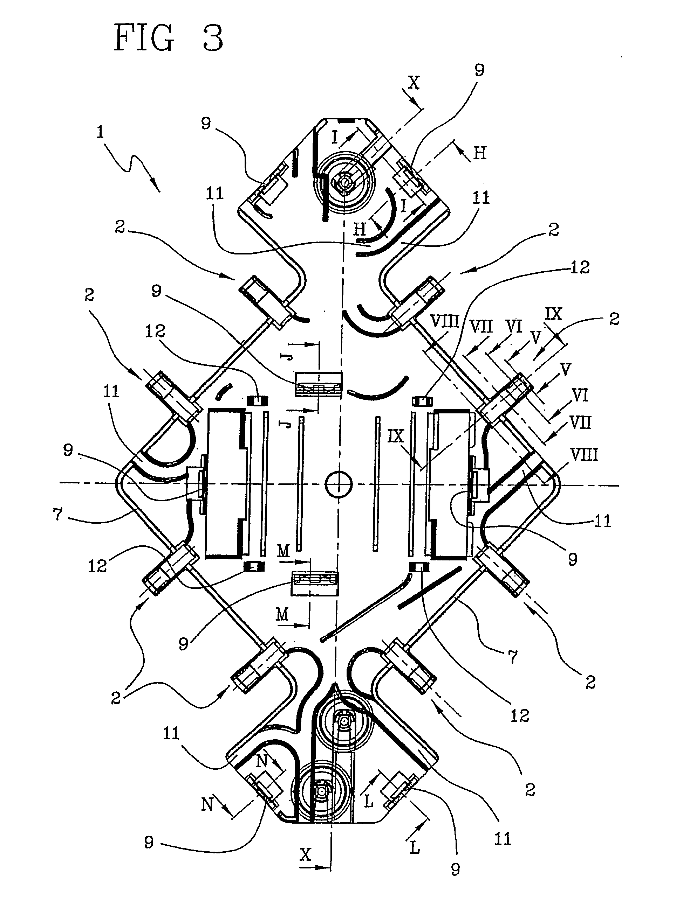

Figure 3 is a plan view of the internal face of figure 1.

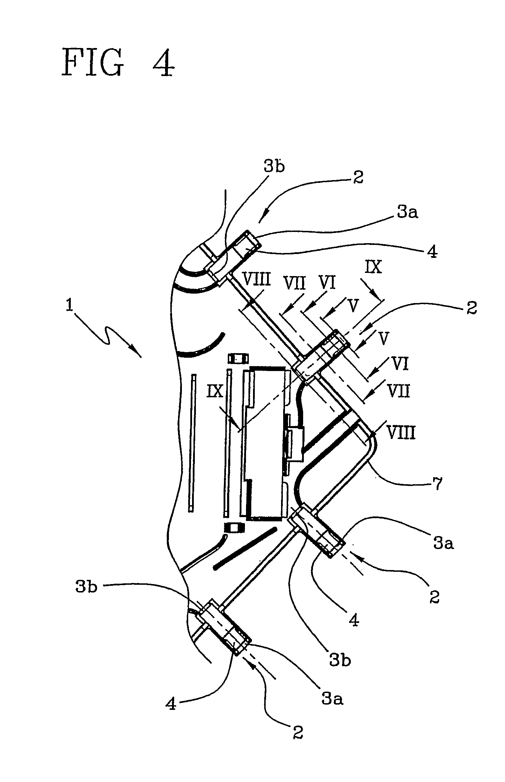

Figure 4 is an enlarged detail of figure 3.

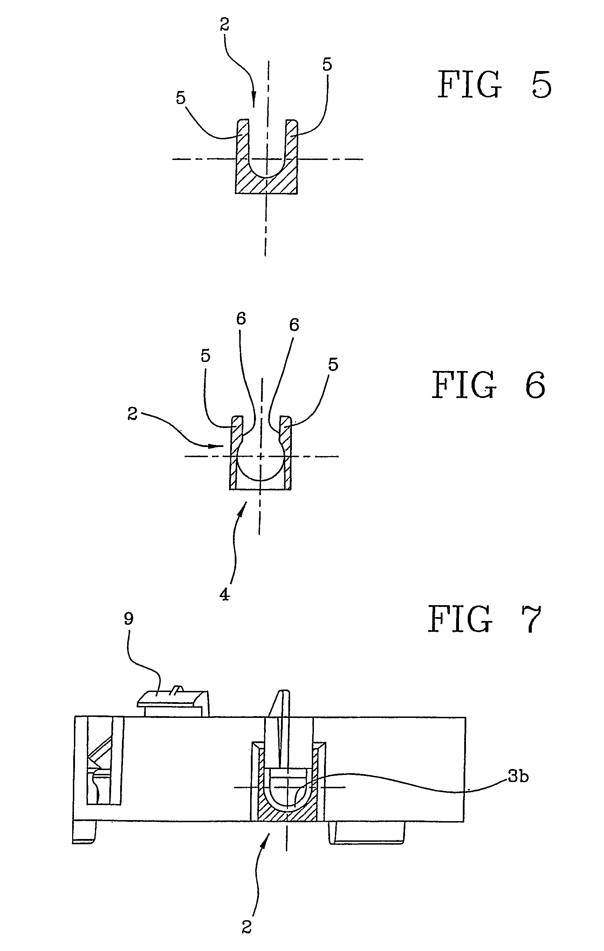

Figure 5 is section V-V of figure 4.
Figure 6 is section VI-VI of figure 4.
Figure 7 is section VII-VII of figure 4.
Figure 8 is section VIII-VIII of figure 4.
Figure 9 is section IX-IX of figure 4.

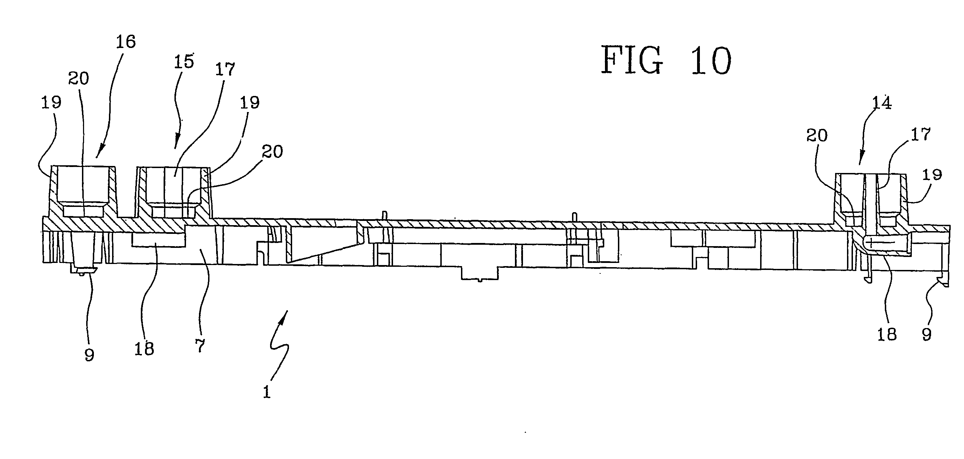

Figure 10 is section X-X of figure 3.

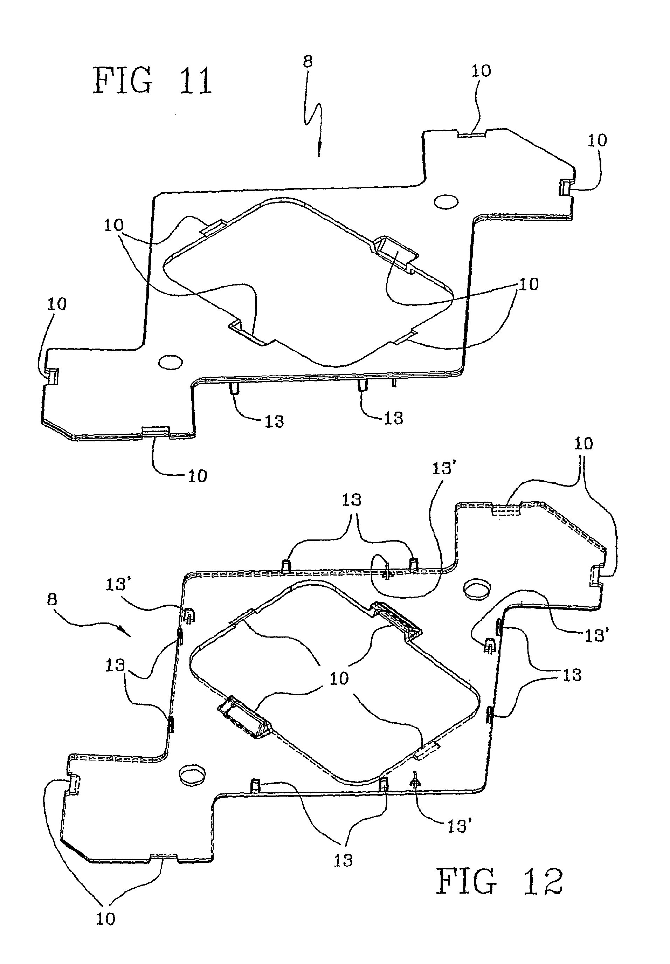

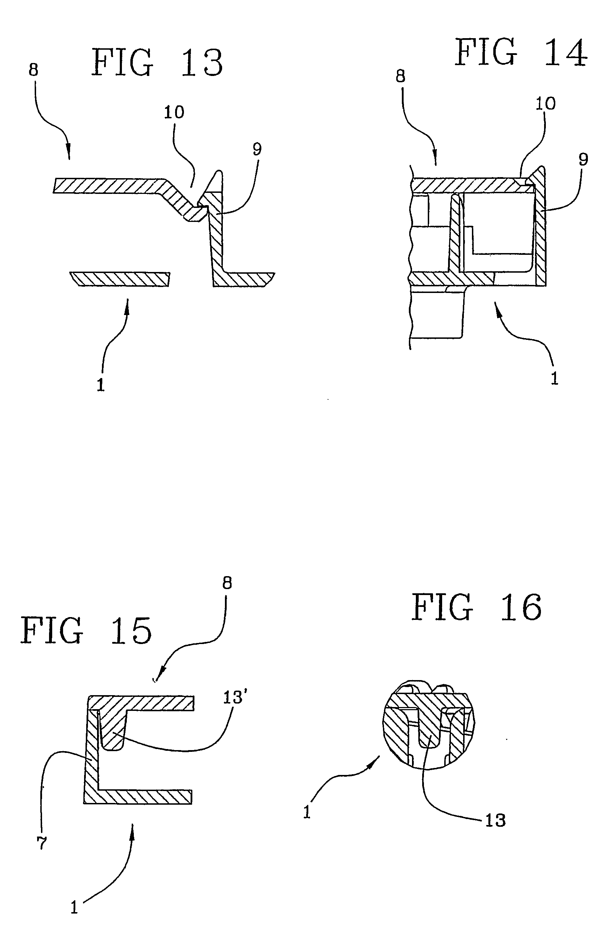

Figure 11 is a perspective view of the upper face of the cover of the support element.
Figure 12 is a perspective view of the lower face of the cover of figure 11.
Figures from 13 to 16 show, in section, four coupling zones between the cover and the base body.
Figure 17 is an apparatus for intensive treatment of renal insufficiency predisposed to receive an integrated module for blood treatment comprising the support element of the preceding figures.
Figure 18 is a diagram of a multifunctional integrated module, able to perform treatments with a pre-infusion of liquid into the extracorporeal blood circuit, operatively connected to the apparatus of figure 17.
Figure 19 is a diagram of another multifunctional integrated module, able to perform a post-infusion, associated to the apparatus of figure 17.

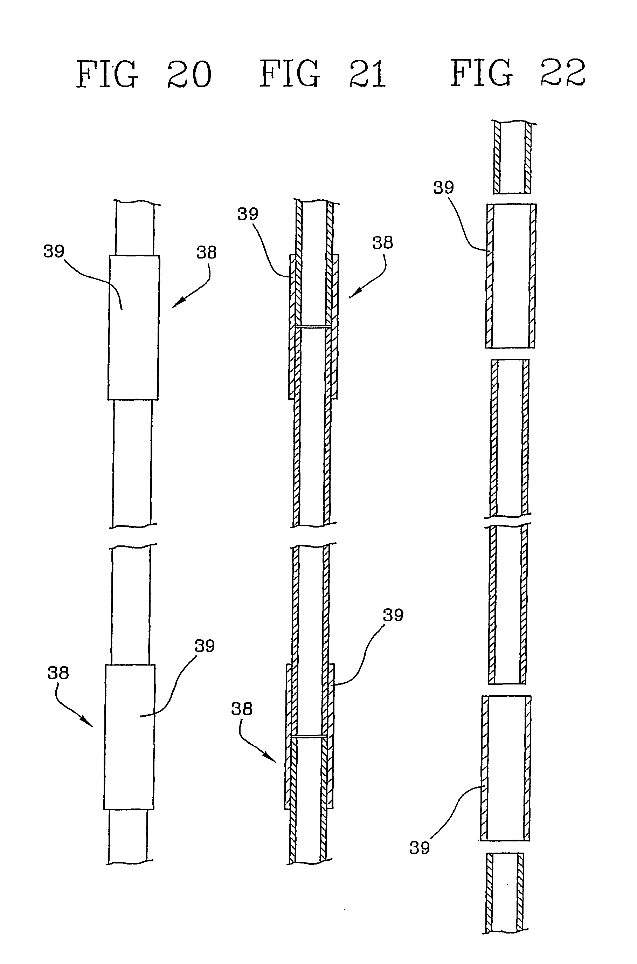

Figure 20 is a tract of a distribution line including a pump segment comprised between two joint collars 39.
Figure 21 is a longitudinal section of figure 20.
Figure 22 is an exploded view of figure 21 in which some components of the distribution line are illustrated before assembly.

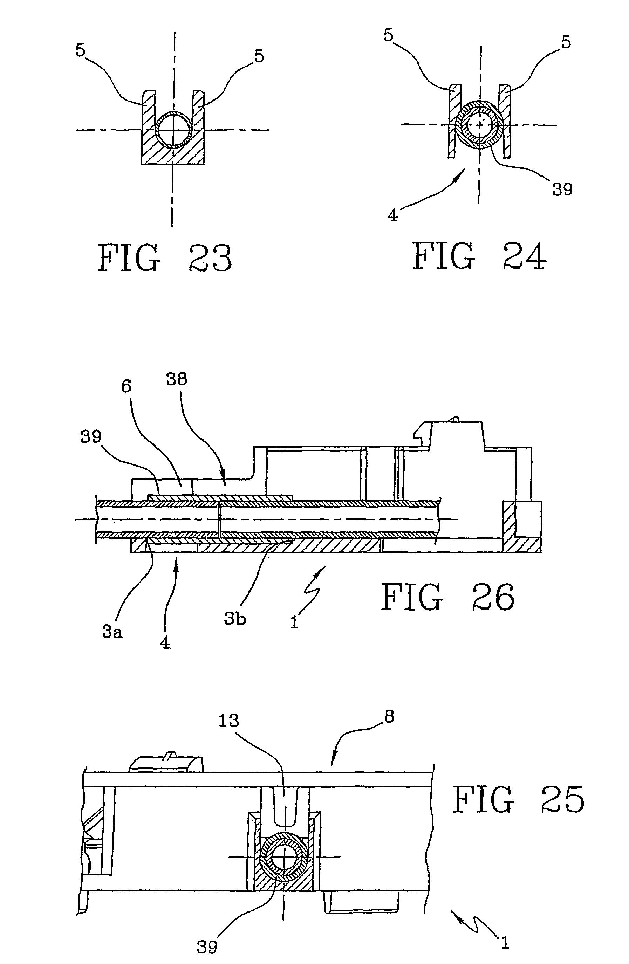

Figures 23, 24, 25 and 26 show figures 5, 6, 7 and 9 with the joint collar 39 coupled in the fixture seating.

### Legend:

- 1: Base body of the integrated module for extracorporeal blood treatment
- 2: Fixture seatings for joint-fixture of line tracts 38
- 3a: Axial reference locator for positioning of a line tract 38 arranged in the fixture seating 2 in an external direction of the base body 1
- 3b: Axial reference locator cooperating with locator 3a and arranged in the fixture seating in an internal direction of the base body 1
- 4: Through-hole located on the bottom of the seating 2 through which an extraction force can be applied from below on the tract of line 38 constrained in the fixture seating 2
- 5: Lateral walls laterally defining the fixture seating 2
- 6: Reliefs developing in an internal direction of the fixture seating 2 from the lateral walls 5
- 7: Raised edge rising from the perimeter of the base body 1
- 8: Cover of the integrated module couplable to the base body 1
- 9: Flexible tabs associated to the base body 1 for fitting the cover 8
- 10: Recesses on the cover 8 cooperating with the tabs 9
- 11: Guide channels associated to the base body 1 for housing two superposed channels of the fluid distribution line
- 12: Joint elements associated to the base body 1 for receiving and constraining the fluid distribution lines
- 13: Teeth projecting downwards from the cover 8 for limiting the raising of the tracts of line 38 constrained in the fixture seatings 2
- 13': Teeth projecting downwards from the cover 8 and situated in proximity or in contact with the internal side of the raised edge 7 for aiding fitting and positioning of the cover 8 on the base body 1
- 14: First connector associated to the base body 1 for mounting the treatment device 37 to the base body 1
- 15: Second connector for mounting the treatment device 3 7
- 16: Third connector for mounting the treatment device 37
- 17: First terminal portion of a connector for fluid connection with the treatment device 37
- 18: Second terminal portion of a connector for fluid connection with a fluid distribution line
- 19: Sealing collar external of the first terminal portion 17
- 20: Connection wall connecting the sealing collar 19 with the first portion 17
- 31: Blood withdrawal line (arterial line)
- 32: Blood return line (venous line)
- 33: Substitution fluid infeed line (pre-infusion line and/or post-infusion line according to the type of integrated module)
- 34: Supply line of a treatment fluid (for example, a dialysis liquid)
- 35: Discharge line of a waste fluid
- 36: Supply line of an anticoagulant fluid
- 37: Blood treatment device (for example a dialysis filter)
- 38: Axial tracts of line with increased external diameter, destined for use in the fixture seatings 2 on the base body 1
- 39: Joint collars causing the increase in diameter of line tracts 38
- 51: Apparatus for extracorporeal blood treatment destined to receive the integrated module
- 52: Housing zone for the integrated module on the apparatus 51
- 53: Peristaltic pumps predisposed on the apparatus 51.

### Detailed Description.

The support element is used as a component of a multifunctional integrated module for extracorporeal blood treatment, in which the integrated module is operatively associable to a multifunctional apparatus for treatment of renal insufficiency. The integrated module is used in particular for intensive treatment of renal insufficiency.

The integrated module comprises the support element, a blood treatment device mounted on the support element, and a complex of fluid distribution lines associated to the support element and cooperating with the treatment device. Each distribution line comprises at least one flexible tube.

### The support element.

With reference to figures from 1 to 16, the following is a description of the support element. It comprises a base body 1 which in turn comprises a part consisting of a flat plate with a plurality of perimeter edges giving the body 1 a polygonal shape. In more detail, the flat plate has a rhomboid central part and two projecting parts, upper and lower, also rhomboid and extending along a common longitudinal axis.

The base body 1 exhibits means for connecting, for receiving and constraining the complex of fluid distribution lines. The means for connecting project from an internal face of the flat plate of the base body 1 and are made in a single piece there-with by press-forming (injection) of a plastic material.

The means for connecting the complex of lines comprise a plurality of fixture seatings 2 located on the periphery of the central rhomboid part of the base body 1. In more detail, the base body 1 exhibits two fixture seatings 2, paired and beside the other, for each side of the rhombus. Each fixture seating 2 is predisposed and configured for a resilient press-fitting and joint-fitting of a corresponding tract of a fluid distribution line, as will better emerge from the following description.

Each fixture seating 2 is axially elongate, in the shape of a superiorly-open channel, for receiving a corresponding axially elongate tract of a fluid distribution line. The housing channel is also open at opposite ends thereof. Each fixture seating 2 is provided with two axial reference locators 3a, 3b, respectively external and internal, axially distanced and opposite, between which the tract of line will be positioned and constrained. Each fixture seating 2 can be provided with a single reference locator 3a or 3b for the positioning of the tract of line in the fixed position.

Each axial locator 3a, 3b, is fashioned from a raised edge projecting towards the inside of the fixture seating 2 at an axial end opening of the fixture seating 2. The raised edge cooperates contactingly with a corresponding projection, in the form of an annular abutment, predisposed externally of the tract of line destined to be engaged in the fixture seating 2, as will be better explained herein below. The coupling between a projection on the tract of line and the corresponding internal raised edge determines a correct and precise positioning of the tract of line in the fixture seating 2.

In more detail, the means for connecting the lines comprise a plurality of pairs of fixture seatings 2; the fixture seatings of each pair are situated side-by-side on the periphery of the base body 1 and receive and constrain two tracts of end of a U-shaped arched segment of a line. Each pair of fixture seatings 2 is arranged on a different perimeter side of the rhomboid central part of the base body 1, each pair having a different orientation with respect to the other pairs. The U-shaped segment cooperates with a peristaltic pump belonging to the treatment apparatus, as will be better explained herein below (the segment is known as the pump segment for this reason). The U-shaped pump segment projects externalwise beyond the periphery of the base body 1.

In more detail, each fixture seating 2 extends axially in length, with a rounded-U-shaped transversal section, to receive an axial tract of a fluid distribution line, and exhibits at two opposite axial ends two undercut surfaces corresponding to two opposite axial directions of the tract of fluid distribution line in the longitudinal seating. The two axial undercut surfaces are fashioned from the two above-mentioned raised edges and result in axial locators 3a and 3b which, as mentioned above, determine the axial positioning of the tract of line in the constrained position thereof.

The upper opening for forced insertion of the tract of line has at least one end part, facing externalwise of the base body 1 (i.e. towards the U-shaped arched segment of the corresponding fluid distribution line), having a passage section which is narrower than the maximum width of the seating: that is, the upper entrance to the channel is narrower with respect to the "real" width of the seating, i.e. the part of the seating where the tract of line is housed when in the constrained position. The tract of line inserted into the channel of the seating has an external diameter which is greater than the minimum width of the passage section, so as to create a friction insertion, and has a diameter which is about equal to the maximum width of the fixture seating, so that the tract of line inserted and fixed in the seating is not crushed, or in any case only very lightly crushed by the walls of the seating.

Each fixture seating 2 inferiorly exhibits a through-hole 4, facing the upper insertion opening, through which a pressure from below can be exerted on the tract of line constrained in the fixture seating in order to extract it (if this is envisaged after use of the integrated module) through the upper insertion opening.

Each fixture seating 2 exhibits an axial end tract which extends axially internally of the perimeter of the base body 1 and an axial end tract which extends externally.

As mentioned above, each fixture seating 2 comprises an upper opening for insertion of a tract of line predisposed for the insertion. At least a part of the length of the upper opening projects externally beyond the perimeter edge of the base body 1. In more detail, the opening is laterally delimited by two lateral walls 5 located side-by-side, each of which walls 5 exhibits an upper edge. The upper opening of the channel is delimited by the upper edges of the two lateral walls 5. In the part of the opening projecting beyond the base body 1, the two upper edges of the lateral walls 5 are straight (and continuous) and parallel one to another, so that the upper opening of the seating extends in a same plane as the lie plane of the upper edges of the lateral walls 5, which are conformed so that the upper opening is flat and extends throughout the length of the seating, or at least for the part thereof which extends beyond the perimeter edge of the base body 1.

Each of the two lateral walls 5 internally exhibits a lateral relief 6 projecting from the wall 5 towards the inside of the opening to define an undercut surface with respect to an extraction direction of the tract of line through the upper opening of the seating: the two reliefs 6, one for each lateral wall 5, face towards and cooperate with each other in order to obstruct the extraction of the tract of line from the channel of the seating.

Each undercut surface, and the lateral relief 6 giving rise to the undercut surface, is predisposed inferiorly of the upper edge of the lateral wall 5; in other words each lateral relief 6 emerges laterally from a side of the lateral wall 5, without emerging upwards, i.e. beyond the upper edge of the wall 5 itself. Each lateral relief 6 is situated in a part of end of the seating facing towards the U-shaped line segment (the pump segment). Each undercut surface is inclined with respect to the extraction direction of the tract of line so that the line can be extracted by force; in effect, the undercut surface lends a certain stability to the positioning of the tract of line with respect to extraction, while the inclination of the undercut surface is such that, by acting with an appropriate extracting force, the tract of line can be removed from its fixture seating.

Each fixture seating 2 exhibits two series of undercut surfaces which operate in two reciprocally transversal directions of movement of the tract of line with respect to the seating: one of the directions is that of axial sliding (in this case the undercut surfaces are situated on the locators 3a and 3b and act in opposite directions) and the other direction is the extraction direction, through the upper opening for forced insertion. The combined action of these undercut surfaces determines the stable and precise positioning of the tract of line in the fixture seating 2.

The internal face of the base body 1, i.e. the face from which the means for connecting for the various lines emerge, exhibits a raised perimeter edge 7 for laterally containing at least a part of the complex of distribution lines.

In other words, the base body 1 comprises a vertical front wall (where front and vertical relate to the work position of the module on the blood treatment apparatus) which comprises the flat plate-shaped part, and a perimeter wall (i.e. the raised edge 7) arranged on the back of the front wall (once more with reference to the work position of the integrated module when mounted on the apparatus). The perimeter wall in effect develops in a distancing direction from the posterior side of the front wall and defines a work seating in which at least a part of the complex of fluid distribution lines can be housed, which lines are destined to be associated to the support element. The height of the raised edge 7 is at least double that of the external diameter of a tube of a fluid distribution line; therefore the work seating can contain two tubes, one above another.

The support element further comprises a cover 8 coupled to the base body 1, which cover 8 is provided for closing at least a part of the complex of distribution lines associated to the means for connecting. The cover 8 at least partially closes the work seating housing the lines. The cover 8 helps keep the fluid distribution lines in a stable position in the work seating. The cover 8, with the module in the work position on the apparatus, is situated behind the front wall of the base body 1.

The base body 1 comprises means for hooking for removably coupling the cover 8 to the base body 1, made in a single piece with the base body 1 itself. A part of the means for hooking is predisposed on the perimeter of the base body 1, while another part thereof is arranged internally of the perimeter. The means for hooking comprise a plurality of flexible tabs 9 which emerge from the base body 1 and which are each provided with an engaging tooth which couples with a recess 10 in the cover 8. The cover 8 exhibits at its centre a through-hole on a rim of which some of the recesses 10 are afforded, while others of the recesses 10 are arranged on the external perimeter.

Various guide channels of the distribution lines emerge from the internal face of the base body 1; these channels are both curved and straight and define pathways followed by the distribution lines. Each channel is defined by two lateral walls, side-by-side and parallel. The reciprocal distance of two side-by-side walls is about equal to an external diameter of the tubes. For some channels, indicated by 11, the lateral walls are configured and arranged in such a way that a height of the channel is at least double a width thereof; these channels 11 are able to house or laterally contain two tracts of line, one above another.

The internal face of the base body 1 also exhibits some pairs of fixture elements 12 for resilient fixture of lines. The fixture elements 12 of each pair cooperate and are arranged one in front of another in a same pair; they hold the distribution lines firm and tight.

The cover 8 closes off at least a part of the internal face bearing the means for connecting the lines. The cover 8 extends in a sort of flat plate shape, with a perimeter that corresponds to the perimeter of the internal face of the base body 1. Thus it comprises a rhomboid central part with two end parts, one upper and the other lower, also rhomboid and arranged along a median longitudinal axis of the cover 8. At a centre thereof the central part of the cover 8 exhibits the above-cited through-hole, which is rectangular in shape. With the cover 8 coupled to the base body 1, a containment space is defined between the internal face of the base body 1 and an internal face of the cover 8, which closes at least a part of the complex of distribution lines.

On a periphery of the central part thereof, the cover 8 exhibits a plurality of teeth 13, projecting downwards, each of which is associated to a fixture seating 2. Each tooth 13, when the cover 8 is coupled to the base body 1, at least partially enters a respective fixture seating and thus limits a raising of the tract of line constrained in the fixture seating 2. When the cover 8 is mounted on the base body 1, the lower end of each tooth 13 is located slightly above the undercut surface on the relief 6 which prevents a raising of the tract of line constrained in the fixture seating 2. Should the tract of line be raised beyond the undercut surface, the teeth 13 provide a guarantee against further raising thereof.

On an external face thereof located opposite to the internal face, the base body 1 also exhibits means for connecting, for attaching a blood treatment device (for example a high-flow dialyzer). The means for connecting are also made in a single piece with the base body 1, by press-forming. The means for connecting the dialyzer comprise a first and a second connector, 14 and 15, associated to the base body 1 and located at a distance one from the other; they are destined to receive and engage with corresponding seatings afforded on the blood treatment device which is mountable on the support element. The first and second connectors 14 and 15 are made in a single piece with the base body 1. There is also a third connector 16, distanced from the first and second connectors 14 and 15 and made in a single piece with the base body 1. The three connectors 14, 15 and 16 define, in combination one with another one, a plurality of pairs of connectors having differentiated interaxes for engaging with corresponding pairs of seatings associated to different blood treatment devices mountable on the support element. The three connectors 14, 15 and 16, are unaligned with one another.

Each connector 14, 15 and 16 defines a fluid passage having a first terminal portion 17, destined to be set in fluid communication with a corresponding channel in a respective seating on the blood treatment device, and a second terminal portion 18, destined to be set in fluid communication with one of the fluid distribution lines associable to the base body 1. The fluid passage is integrated in the connectors 14, 15 and 16, which are in turn made in a single piece with the base body 1.

In more detail, each connector 14, 15, 16 comprises a tubular channel, which defines the first terminal portion 17, a sealing collar 19, located in a radially external position to the tubular channel, and a connecting wall 20 which develops continuously between an external lateral surface of the tubular channel and an internal lateral surface of the sealing collar 19, defining an annular seating for engaging each seating. The tubular channel is coaxially arranged with respect to the sealing collar 19. The annular seating exhibits a bottom which is delimited by the connecting wall 20. The annular seating exhibits an increasing radial dimension as it progresses from the bottom connecting wall 18; it comprises a first zone, adjacent to the bottom and having a constant radial dimension; a second zone, distal with respect to the bottom and having a constant radial dimension which is greater than the radial dimension of the first zone; and a third zone, which is a transit zone between the first and second zones and which has a progressively growing radial dimension as it progresses away from the bottom connecting wall 20.

Each connector 14, 15 and 16, is directly constrained to the base body 1.

The tubular channel, i.e. the channel defining the first terminal portion 17, and the sealing collar 19 of each seating 14, 15 and 16, are parallel to one another in the base body 1, defining a single coupling direction with the corresponding connectors of a treatment device.

The base body 1 and the connectors 14, 15 and 16 of the blood treatment device (located on the external face of the base body 1) are made of a rigid material in order to offer a good mechanical support for the device.

### The complex of fluid distribution lines.

The fluid distribution lines comprise flexible tubes having internal sections for fluid passage which internal sections are the same for all the tubes.

The complex of fluid distribution lines associated to the support element comprises: a blood withdrawal line 31, a blood return line 32, a substitute fluid infeed line 33, a treatment fluid infeed line 34 (for example a dialysis liquid), a waste fluid discharge line 35, and an anticoagulant infeed line 36.

37 denotes a blood treatment device mounted on the support element and comprising a first and a second chamber, separated from each other by a semi-permeable membrane. The blood treatment device 37 is selected from a group comprising devices for: hemofiltration, hemodialysis, high-flow dialyzers and hemodiafiltration devices. In the illustrated embodiment the treatment device is a high-flow dialyzer.

The blood withdrawal line 31 is connected to the first chamber of the treatment device 37. The blood return line 32 receives the treated blood exiting from the first chamber and returns it to the patient. The treatment fluid infeed line 34 is fluidly connected to an inlet of the second chamber of the blood treatment device 37. The treatment fluid (dialysis liquid) is destined to receive, through the semi-permeable membrane, the impurities present in the patient's blood and the excess fluid which is to be removed from the blood. The waste fluid discharge line 35 is fluidly connected to an outlet of the second chamber and carries the waste fluid exiting from the blood treatment device 37 to a collection recipient.

The blood treatment device 37 comprises a blood withdrawal port, a blood return port, a treatment fluid inlet port and a waste fluid discharge port, in fluid connection (respectively) with the blood withdrawal line 31, the blood return line 32, the treatment fluid supply line 34 and the waste fluid discharge line 35.

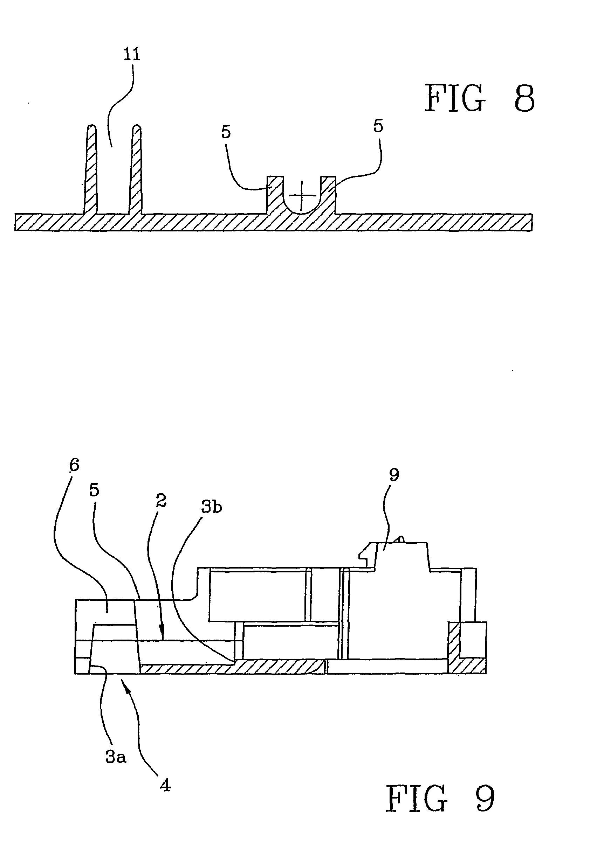

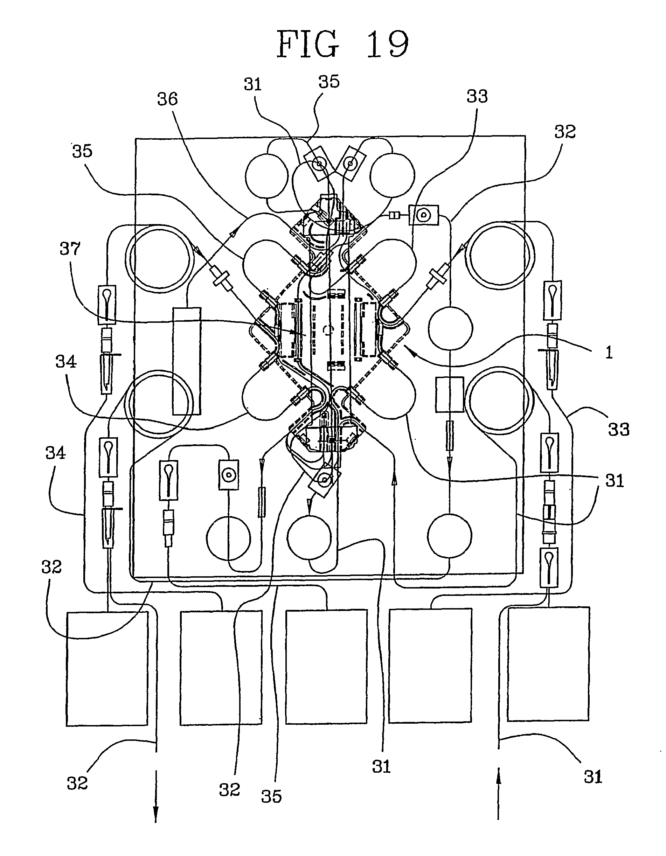

The substitution fluid infeed line 33 receives an infusion or substitution fluid from a source (for example a tank or bag) and feeds it through a Y connection to a blood circulation line; or to the blood withdrawal line 31 (pre-infusion upstream of the blood treatment device 37, as in the example of figure 8) or to the blood return line 32 (post-infusion downstream of the blood treatment device 37, as in the example of figure 19).

The anticoagulant supply line 36 infeeds an anticoagulant into the blood withdrawal line 31 through a Y connection.

The blood withdrawal line 31, the substitution fluid infeed line 33, the treatment fluid infeed line 34, the waste fluid discharge line 35, each exhibit at least two tracts 38 having a predetermined length and an increased external diameter with respect to adjacent tracts thereof. The two tracts 38 having increased external diameter are arranged on the respective line at a predetermined distance one from the other. Each axial tract 38 having an increased external diameter gives rise to two external abutments. Each axial tract 38 with increased external diameter comprised between the two abutments will be press-inserted in a corresponding fixture seating 2 arranged on the periphery of the base body 1. When the complex of distribution lines is applied to the support element, each segment of line comprised between two axial tracts 38 having an increased external diameter (the pump segment) is arranged in a U-shaped arch, and is coupled to a peristaltic pump belonging to the treatment device, which pump invokes circulation of fluid in the line. The length of the segment of line comprised between the two tracts of line 38 (pump segment) is predetermined in order to guarantee a correct coupling with the peristaltic pump.

In more detail, each axial tract of line 38 with an increased external diameter comprises a joint collar 39 which contains and coaxially joins two end parts and two adjacent tracts of line: a part of an end of a tract of line is inserted into the collar 39 up to not more than half of the length of the collar 39 itself, through an axial opening in the collar 39, while the part of end of the other tract of line is inserted into the axial hole at the other end of the collar 39. A stable joint of the collar 39 with the end parts of the tracts of line can be achieved by known means, for example by hot-welding. The inter diameter of the joint collar 39 is about the same as the external diameter of the tubes forming the distribution lines. The U-shaped segment (pump segment) comprises a tube having two opposite ends inserted in two joint collars 39 and in fluid connection with two ends of two tubes, also inserted, but on the opposite side, in the same joint collars. The material used for making the arched segments of tube is suitable for operation with the peristaltic pumps and is different to the material used for the other two tubes coupled to the joint collar 39, which undergo no interaction with the peristaltic pumps. Each joint collar 39 is made of a more rigid material that that of the tracts of line the collar 39 joins. During the assembly stage of the integrated module the collar 39 is inserted snugly into the fixture seating 2. The axial length of the joint collar 39 is about the same as the axial distance between the axial locators 3a and 3b located in the fixture seating 2. The collars 39 can be slightly longer or shorter, depending on whether the desired coupling between the collar 39 and the seating 2 is achieved by axial interference or with axial play. The external diameter of the collar 39 is about the same as or slightly smaller than the maximum width of the fixture seating 2; the diameter is also greater than the minimum width of the upper insertion opening of the fixture seating 2, so that the insertion of the joint collar 39 in the fixture seating 2 is achieved by resilient friction fitting.

As previously mentioned, the length of the segment of line comprised between two joint collars 39 (pump segment) is predefined in order to obtain, once the line is coupled to the support element, a U-shaped segment precisely positioned and shaped for interaction with the peristaltic pump.

The arrangement of the complex of distribution lines on the integrated module is described in more detail herein below.

### The integrated module.

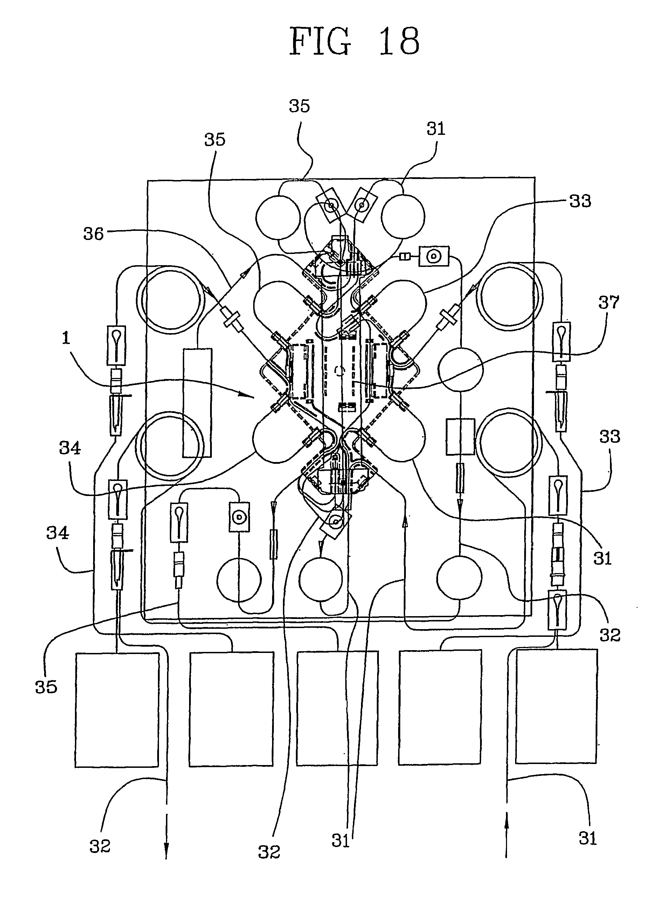

Figures 18 and 19 show two integrated modules which are different essentially because of the different configurations of the distribution lines. In more detail, in the first of the modules (figure 18), the substitution fluid infeed line 33 is inserted into the blood withdrawal line 31 (pre-infusion), while in the second module (figure 19) the substitution fluid infeed line 33 (the same numbers are used in the two figures for the sake of simplicity) is inserted into the blood return line 32 (post-infusion).

### The extracorporeal blood treatment apparatus.

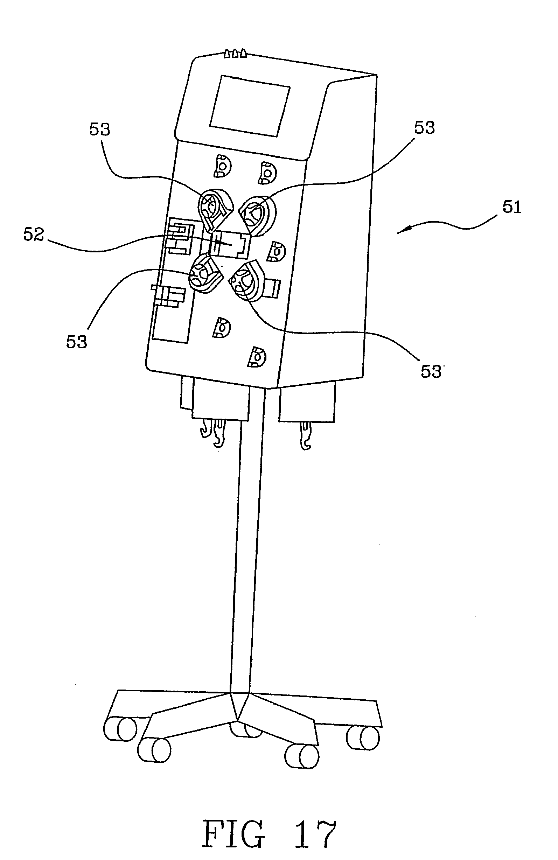

The extracorporeal blood treatment apparatus, illustrated in figure 17 and indicated in its entirety by 51, comprises a housing zone 52 predisposed for receiving the integrated module for extracorporeal blood treatment (selectively one of the two above-described modules); the four peristaltic pumps 53 are located by the side of the housing zone 52 and are operatively associated to the four U-shaped segments of the fluid distribution lines in the integrated module. The apparatus can be used to perform treatments requiring the use of fewer than four pumps, in cooperation with appropriate modules provided with fewer than four U-shaped segments. The apparatus 51 further comprises a central treatment control unit, of known type and not illustrated, which controls the various treatment procedures. No special explanation of these is necessary in the present description.

The axial locators 3a and 3b ensure the precision in position and stability of the U-shaped segment cooperating with the peristaltic pumps 53. One locator alone, either external 3a or internal 3b according to the rotation direction of the corresponding pump 53, can be provided for each fixture seating 2; the locator will operate contrastingly to the action of the pump in relation to the tract of line constrained in the fixture seating 2, which action can be drawing or thrusting according to the direction of the pump and the position of the fixture seating 2 (if the seating 2 is located upstream of the pump the tube housed in the seating is drawn by the pump, while if the seating 2 is located downstream of the pump the tube is thrust). Both locators 3a and 3b can be provided in one alone of the two fixture seatings 2 located at the ends of a U-shaped segment (pump segment); and one locator 3a, 3b alone can be provided in one alone of the two fixture seatings 2 located at an end of a U-shaped segment.

The lateral reliefs 6, which define a narrow-section upper inlet of the fixture seating, are also undercuts which hold the tract of line engaged in the fixture seating 2 in position.

### Integrated module assembly.

The assembly of the integrated module for fluid treatment comprises the following stages:
manufacture of the support element, for example by press-forming the plastic material of the two pieces which make up the element: the base body 1 and the cover 8;
fixing the blood treatment device to the support element, in particular to the external face of the base body 1;
associating the complex of distribution lines for the fluids to the support element and to the treatment device.

Fitting the treatment device to the support element includes the following stages:
- Selecting a pair of connectors from connectors 14, 15 and 16, to which the seatings on the treatment device are to be fixed;
- Depositing a predetermined quantity of glue on the annular seatings of each selected connector;
- At least partially inserting each seating in the respective annular seating in order to achieve a mechanical lock and a liquid-sealed coupling; during the insertion stage, at least a portion of the predetermined quantity of glue enters the second zone of the annular seating, i.e. the upper broadened zone which is radially larger than the bottom zone of the annular seating; on conclusion of the insertion stage, the volume of the glue added to the volume of the portion of seating housed in the annular seating is lower than the overall volume of the annular seating, in order to avoid any glue exiting from the seating and occupying even minimally the fluid passage zone.

The coupling of the complex of fluid distribution lines to the support element comprises an insertion stage of the junction collars 39 internally of the fixture seatings 2. This stage is performed by a simple pressure fit, taking care to make sure the abutments formed by each junction collar 39 meet with the locators 3a and 3b of the respective fixture seating 2. The junction collar 39 is friction-fitted in the fixture seating 2 through the upper opening defined between the lateral walls 5, which opening is narrower than the diameter of the junction collar 39. The junction collar 39, which externally identifies the axial tract of line engaged in the fixture seating 2, is thus stably joint-coupled in the seating 2, with no need for glues and with considerable precision of positioning thanks to the coupling between the locators 3a and 3b and the ends of the junction collar 39.

The cover 8 is coupled to the base body 1 after the distribution lines have been engaged. The cover 8 at least partially closes off the lines engaged to the base body 1 and guarantees the lines' housed stability.

The integrated module is destined to be replaced by a new module. The support element can be easily separated from the distribution lines and the treatment device and re-used, after suitable washing and sterilizing procedures, as a support element for a new treatment module; or it can be disposed of suitably.

Detaching the distribution lines is done by removing the cover 8 and extracting the complex of lines from the housing zone on the base body 1: the extraction procedure is made easier by the through-holes 4 which enable an extracting pressure to be exerted from the bottom on the collars 39, causing the lines to exit through the upper openings of the fixture seatings 2.

## Claims

1. A series of two elements destined to be coupled one to another, wherein the first element is a support element for an integrated module for extracorporeal blood treatment and the second element is a complex of distribution lines for fluids, the support element comprising:
- a base body (1);
- at least one fixture seating (2) located on the base body (1) and axially extended for housing an axially extended tract (38) of a fluid distribution line;
- at least one axial locator (3a, 3b) arranged in the said fixture seating (2) for positioning the said axial tract of line in a fixed position, the said at least one axial locator (3a, 3b) being destined to interact with a corresponding element (39) predisposed on the said axial tract (38) of line; and
the complex of distribution lines comprising:
- at least one line having at least one tract (38) of line which has a larger external diameter than adjacent tracts thereof, giving rise to two external abutments; the at least one tract (38) with larger diameter comprised between the two external abutments being destined for insertion in the fixture seating (2) predisposed on the support element, **characterised in that** each tract (38) having a larger external diameter comprises a junction collar (39) which contains and joins two end zones of two parts of fluid-connected line.

2. The series of claim 1, wherein the support element comprises two axial locators (3a, 3b), axially reciprocally distanced, between which the axial tract of line is positioned in a fixed position.

3. The series of claim 1 or 2, wherein the fixture seating (2) comprises a superiorly-open channel for insertion of the axial tract of line and wherein the said at least one axial locator (3a, 3b) comprises an edge projecting internally of the channel from a wall delimiting the channel.

4. The series of any one of the preceding claims, wherein the support element comprises at least one pair of the fixture seatings (2) arranged side-by-side on a periphery of the base body (1), the at least one pair of fixture seatings receiving two tracts of end of a U-shaped segment of at least one line, the U-shaped segment being destined to cooperate with a peristaltic pump (53).

5. The series of claim 4, wherein the U-shaped segment projects externally beyond the periphery of the base body (1).

6. The series of claim 4 or 5, wherein the base body (1) has a part which is flat plate-shaped with a plurality of perimeter sides which define a polygonal shape of the base body (1) and wherein the base body (1) exhibits a plurality of pairs of fixture seatings (2), each pair thereof being made up of two fixture seatings (2) arranged side-by-side and reciprocally parallel and arranged on one of the plurality of perimeter sides.

7. The series of any one of the preceding claims, wherein the axial locator (3a, 3b) comprises a surface which is undercut with respect to an axial direction of the axial tract of line.

8. The series of any one of the preceding claims, wherein the fixture seating (2) is predisposed and configured for forced insertion and resilient joint-coupling of the axial tract of line, the fixture seating (2) having at least one opening for insertion of the axial tract of line, a passage section of the at least one opening being at least partially smaller in width than a maximum width of the fixture seating (2).

9. The series of any one of the preceding claims, wherein the fixture seating (2) is delimited by a surface for receiving the axial tract of line, which surface exhibits at least two undercut surfaces predisposed and configured for obstructing displacements of the axial tract of line with respect to the fixture seating (2) in two reciprocally transversal displacement directions, namely an axial direction and an upwards extraction direction passing through an upper insertion opening.

10. The series of any one of the preceding claims, wherein the fixture seating (2) superiorly exhibits an opening for forced insertion of the axial tract of line and inferiorly exhibits at least one through-hole (4), facing the insertion opening and through which a pressure can be exerted from below on the axial tract of line when engaged in the fixture seating (2), enabling extraction of the axial tract of line through the superior opening for forced insertion.

11. The series of any one of the preceding claims, wherein:
- the fixture seating (2) is defined by a channel having a superior opening for insertion of the axial tract of line;
- the channel extends axially and at least partially axially projects in an externalwise direction beyond a perimeter border of the base body (1);
- the channel is laterally delimited by two walls (5) arranged side-by-side, each of which two walls (5) exhibits an upper edge;
- the insertion opening is delimited by the upper edges of the two lateral walls (5);
- at least one lateral relief (6) projects from at least one of the two lateral walls (5) and projects inwardly of the channel; the at least one lateral relief (6) defining an undercut surface with respect to an extraction direction of the axial tract of line through the superior opening of the channel;
- the undercut surface is predisposed inferiorly of the upper edge of the lateral wall (5).

12. The series of claim 11, wherein the support element comprises two lateral reliefs (6), one for each lateral wall (5), which two lateral reliefs face one
another and cooperate to obstruct an extraction of the axial tract of line.

13. The series of claim 11 or 12, wherein the channel is destined to constrain a tract of end of a segment of U-shaped line, operatively associable to a peristaltic pump (53), and wherein each lateral relief (6) is situated in a part of end of the channel which part of end is facing towards the U-shaped segment.

14. The series of any one of the claims from 11 to 13, wherein each undercut surface is inclined with respect to an extraction direction of the axial tract of line to enable extraction of the axial tract of line by a forcing movement.

15. The series of any one of the preceding claims, wherein the base body (1) is flat-plate shaped and the fixture seatings (2) for the complex of distribution lines extend over an internal face thereof, and wherein the support element further comprises a cover (8) removably couplable to the base body (1) and destined to cover at least a part of the internal face which bears the fixture seatings (2); when the cover (8) is coupled to the base body (1), a containment space for at least a part of the complex of distribution lines is defined between the internal face of the base body (1) and an internal face of the cover (8).

16. The series of claim 15, wherein the cover (8) exhibits at least one tooth (13) projecting downwards which, when the cover (8) is coupled on the base body (1), enters at least partially in the fixture seating (2) and limits a raising movement of the axial tract line constrained in the fixture seating (2).

17. The series of claim 15 or 16, wherein the internal face of the base body (1) exhibits on a perimeter thereof a raised edge (7) for laterally containing at least a part of the complex of distribution lines, and wherein the cover (8) is flat plate-shaped with a perimeter corresponding to the perimeter of the internal face of the base body (1).

18. The series of claim 17, wherein the base body (1) comprises at least one hooking tab (9) made in a single piece with the base body (1) and destined to removably couple the cover (8) to the base body (1).

19. The series of any one of the preceding claims, wherein the fixture seating (2) exhibits an axial end tract which extends axially internally of the perimeter side of the base body (1) and an end tract which extends axially externally of the perimeter side of the base body (1).

20. The series of one of the preceding claims, wherein a stable joint of the junction collar (39) with the two end zones of the two parts of line is achieved by hot-welding.

21. The series of claim 1, comprising at least two of the at least one tract (38) of line with a larger external diameter than adjacent tracts thereof, which at least two tracts of line are axially distanced one from another; a segment of line being comprised between the at least two tracts of line, which segment of line is made to form a U-shaped arched segment which is operatively associated to a peristaltic pump (53).

22. The series of claim 21, wherein the material used for making the U-shaped arched segments is suitable for operation with the peristaltic pumps and is different from the material used for the two end zones of the parts of line connected by means of the junction collar (39).

23. The series of claim 1, wherein the junction collar (39) is made of a more rigid material than the two end zones of the parts of line connected by means of the junction collar (39).

24. An assembly process of the series of elements of claim 1, wherein the tract of line (38) having a larger diameter and comprised between the two abutments is inserted in the fixture seating (2) predisposed on the support element, the two abutments coinciding with the axial locator (3a, 3b) predisposed in the fixture seating (2), the support element being provided with a cover (8) which is subsequently coupled to the base body (1) in order to cover at least partially the tract of line (3 8) having a larger diameter.

25. An integrated module for extracorporeal blood treatment, comprising:
a series made according to any one of claims from 1 to 23;
at least one blood treatment device mounted on the support element comprising at least a first chamber and at least a second chamber which are separated by at least one semi-permeable membrane, wherein
the complex of fluid distribution lines is associated to the support element and cooperating with the blood treatment device.

26. The integrated module of claim 25, wherein the complex of fluid distribution lines comprises at least one line made according to any one of claims from 20 to 23.

27. The module of any one of claims from 25 to 26, wherein the support element comprises at least a first and at least a second connector (14, 15) associated to the base body (1) and distanced one from another, destined to receive and constrain corresponding seatings of a blood treatment device which is mountable on the support element;
the blood treatment device is fixed to the base body (1) and is fixed by at least one pair of connectors from the first, second and third connectors (14, 15, 16); and wherein
the blood treatment device further comprises:
- a containment body;
- at least one semi-permeable membrane operating internally of the containment body and defining a first chamber and a second chamber;
- a first and a second counter-connector, associated to the containment body and fixed to first, second or third connectors (14, 15, 16) associated to the base body (1), the first connector (14) and the second connector (15) being set in fluid connection with the second chamber of the blood treatment device and with respective first terminal portions (17) of the first connector (14) and the second connector (15);
- at least one inlet port to the first chamber, and at least one outlet port from the first chamber; and wherein
the complex of fluid distribution lines comprises at least one discharge line of waste fluid (35) set in communication with the second terminal portion (18) of one of the first, second and third connectors (14, 15, 16).

28. The module of claim 27, wherein at least one of the lines is constrained to the support element, defining at least one U-shaped arched segment on the support element and being destined to cooperate in use with a peristaltic pump (53).

29. The module of claim 28, wherein the U-shaped segment extends externally with respect to the perimeter wall of the support element.

30. The integrated module of any one of claims from 25 to 29, for use in treatment of renal insufficiency, wherein the blood treatment device is selected from a group comprising hemofiltration, hemodialysis, high-flow filtration and hemodiafiltration devices.

## Patentansprüche

1. Anordnung von zwei Elementen zur Verbindung miteinander, worin das erste Element ein Tragelement für ein integriertes Modul zur extrakorporalen Blutbehandlung und das zweite Element eine Gruppe von Verteilungsleitungen für Fluide ist, wobei das Tragelement folgendes umfasst:
- einen Basiskörper (1);
- mindestens ein Befestigungssitz (2), die am Basiskörper (1) angeordnet ist und sich axial zur Aufnahme eines sich axial erstreckenden Abschnitts (38) einer Fluidverteilungsleitung erstreckt;
- mindestens einen axialen Paßstift (3a, 3b), der in der benannten Befestigungssitz (2) zum festen Positionieren des benannten axialen Leitungsabschnitts angeordnet ist, wobei der mindestens eine axiale Paßstift (3a, 3b) zur Zusammenwirkung mit einem entsprechenden, am benannten axialen Leitungsabschnitt (38) angeordneten Element (39) vorgesehen ist; und
wobei die Gruppe von Verteilungsleitungen folgendes umfasst:
- mindestens eine Leitung mit mindestens einem Leitungsabschnitt (38), der einen größeren Außendurchmesser aufweist als nebenliegenden Abschnitte davon, wodurch zwei Außenanschläge entstehen; der mindestens eine Abschnitt (38) mit größerem Durchmesser zwischen den beiden Außenanschlägen zur Einführung in den am Tragelement angeordneten Paßstift (2) vorgesehen ist, **dadurch gekennzeichnet, daß** jeder Abschnitt (38) mit einem größeren Außendurchmesser ein Anschlussbund (39) umfasst, die zwei Endbereiche von zwei Teilen einer Fluidverbindungsleitung enthält und verbindet.

2. Anordnung nach Anspruch 1, worin das Tragelement zwei axiale Paßstifte (3a, 3b) umfasst, die mit einem axialen Abstand voneinander sind und zwischen den der axiale Leitungsabschnitt fest positioniert ist.

3. Anordnung nach Anspruch 1 oder 2, worin der Befestigungssitz (2) einen oben geöffneten Kanal zur Einführung des axial Leitungsabschnitts umfasst, und worin der mindestens eine axiale Paßstift (3a, 3b) eine Kante umfasst, die in den Kanal von einer den Kanal begrenzenden Wand vorspringt.

4. Anordnung nach irgendeinem der vorherigen Ansprüche, worin das Tragelement mindestens ein Paar von Befestigungssitze (2) umfasst, die nebeneinander auf einem Umfang des Basiskörpers (1) angeordnet sind, wobei das mindestens eine Paar von Befestigungssitze zwei Endabschnitte eines U-förmigen Segments von mindestens einer Leitung empfängt, wobei das U-förmige Segment zur Zusammenwirkung mit einer peristaltischen Pumpe (53) vorgesehen ist.

5. Anordnung nach Anspruch 4, worin das U-förmigen Segment nach außen über den Umfang des Basiskörpers (1) vorspringt.

6. Anordnung nach Anspruch 4 oder 5, worin der Basiskörper (1) einen Teil hat, der als eine flache Platte ausgebildet ist, mit einer Vielzahl von Umfangsseiten, die eine polygonale Gestalt des Basiskörpers (1) definieren, und worin der Basiskörper (1) eine Vielzahl von Paaren von Befestigungssitze (2) aufweist, wobei jedes Paar davon aus zwei nebeneinander angeordneten Befestigungssitze (2) besteht, die parallel zueinander sind und auf einer der Vielzahl von Umfangsseiten angeordnet sind.

7. Anordnung nach irgendeinem der vorherigen Ansprüche, worin der axiale Paßstift (3a, 3b) eine Fläche umfasst, die gegenüber einer axialen Richtung des axialen Leitungsabschnitts untergeschnitten ist.

8. Anordnung nach irgendeinem der vorherigen Ansprüche, worin der Befestigungssitz (2) angeordnet und ausgestaltet ist zur Zwangseinführung und elastischen Drehkopplung des axialen Leitungsabschnitts, wobei der Befestigungssitz (2) mindestens eine Öffnung zur Einführung des axialen Leitungsabschnitts besitzt, wobei einen Durchgangsquerschnitt der mindestens einen Öffnung mindestens teilweise eine kleinere Breite hat als eine maximale Breite der Befestigungssitz(2).

9. Anordnung nach irgendeinem der vorherigen Ansprüche, worin der Befestigungssitz (2) durch eine Fläche zum Empfang des axialen Leitungsabschnitts begrenzt ist, welche Fläche mindestens zwei Unterschnittfläche aufweist, die so angeordnet und ausgestaltet sind, um Verstellung des axialen Leitungsabschnitts gegenüber der Befestigungssitz (2) in zwei zueinander quer liegenden Verstellungsrichtungen zu sperren, d.h. in einer axialen Richtung und in einer Ausziehrichtung nach oben durch eine obere Einführungsöffnung.

10. Anordnung nach irgendeinem der vorherigen Ansprüche, worin der Befestigungssitz (2) oben eine Öffnung zur Zwangseinführung des axialen Leitungsabschnitts und unten mindestens ein Durchgangsloch (4) aufweist, das der Einführungsöffnung zugewandt ist und durch das ein Druck von unten auf den axialen Leitungsabschnitt ausgeübt werden kann, wenn er in der Befestigungssitz (2) eingreift, wodurch das Ausziehen des axialen Leitungsabschnitts durch die obere Öffnung zur Zwangseinführung ermöglicht wird.

11. Anordnung nach irgendeinem der vorherigen Ansprüche, worin:
- der Befestigungssitz (2) durch einen Kanal mit einem oberen Öffnung zur Einführung des axialen Leitungsabschnitts definiert ist;
- der Kanal sich axial erstreckt und axial in einer Richtung nach außen über einen Umfangsrand des Basiskörpers (1) mindestens teilweise vorspringt,
- der Kanal durch zwei nebeneinander angeordneten Wände (5) begrenzt ist, wobei jede der beiden Wände (5) eine obere Kante aufweist;
- die Einführungsöffnung durch die oberen Kanten der beiden Seitenwände (5) begrenzt ist;
- mindestens eine Seitenerhebung (6) von mindestens einer der beiden Seitenwände (5) und in den Kanal vorspringt; wobei die mindestens eine Seitenerhebung (6) eine Unterschnittfläche gegenüber einer Ausziehrichtung des axialen Leitungsabschnitts durch die obere Öffnung des Kanals definiert;
- die Unterschnittfläche unter der oberen Kante der Seitenwand (5) angeordnet ist.

12. Anordnung nach Anspruch 11, worin das Tragelement zwei Seitenerhebungen (6), eine für jede Seitenwand (5), umfasst, welche zwei Seitenerhebungen zueinander zugewandt sind und zum Sperren des Ausziehen des axialen Leitungsabschnitts zusammenwirken.

13. Anordnung nach Anspruch 11 oder 12, worin der Kanal zur Verbindung eines Endabschnitts eines U-förmigen Segments, das mit einer peristaltischen Pumpe (53) operativ verbunden werden kann, vorgesehen ist, und worin jede Seitenerhebung (6) in einem Endteil des Kanals liegt, welcher Endteil dem U-förmigen Segment zugewandt ist.

14. Anordnung nach irgendeinem der Ansprüche 11 bis 13, worin jede Unterschnittfläche gegenüber einer Ausziehrichtung des axialen Leitungsabschnitts geneigt ist, um das Ausziehen des axialen Leitungsabschnitts durch eine Zwangsbewegung zu erlauben.

15. Anordnung nach irgendeinem der vorherigen Ansprüche, worin der Basiskörper (1) als eine flache Platte ausgebildet ist und der Befestigungssitze (2) für die Gruppe von Verteilungsleitungen sich auf einer Innenfläche davon erstrecken, und worin das Tragelement eine Abdeckung (8) weiter umfasst, die mit dem Basiskörper lösbar gekoppelt werden kann und zur Abdeckung mindestens eines Teils der Befestigungssitze (2) tragenden Innenfläche vorgesehen ist; wenn die Abdeckung (8) mit dem Basiskörper (8) gekoppelt ist, wird ein Aufnahmeraum für mindestens einen Teil der Gruppe von Verteilungsleitungen zwischen der Innenfläche des Basiskörpers (1) und einer Innenfläche der Abdeckung (8) definiert.

16. Anordnung nach Anspruch 15, worin die Abdeckung (8) mindestens eine Zahn (13) aufweist, die sich nach unten vorspringt und - wenn die Abdeckung (8) am Basiskörper (1) befestigt ist - in der Befestigungssitz (2) mindestens teilweise eindringt, um eine Hebebewegung des in der Befestigungssitz (2) axialen Leitungsabschnitts zu beschränken.

17. Anordnung nach Anspruch 15 oder 16, worin die Innenfläche des Basiskörpers (1) an einem Umfang davon eine erhobene Kante (7) aufweist, um mindestens einen Teil der Gruppe von Verteilungsleitungen seitlich zu enthalten, und worin die Abdeckung (8) als eine flache Platte mit einem Umfang entsprechend dem Umfang der Innenfläche des Basiskörpers (1) ausgebildet ist.

18. Anordnung nach Anspruch 17, worin der Basiskörper (1) mindestens eine Hakenlappe (9) umfasst, die einstückig mit dem Basiskörper (1) hergestellt wird und zur lösbaren Kopplung der Abdeckung (8) mit dem Basiskörper (1) vorgesehen ist.

19. Anordnung nach irgendeinem der vorherigen Ansprüche, worin der Befestigungssitz (2) einen axialen Endabschnitt, der sich axial in der Umfangsseite des Basiskörpers (1) erstreckt, und einen Endabschnitt, der sich axial außerhalb der Umfangsseite des Basiskörpers (1) erstreckt, aufweist.

20. Anordnung nach einem der vorherigen Ansprüche, worin eine feste Kupplung der Anschlussbund (39) mit den beiden Endbereichen der beiden Leitungsabschnitte durch Warmschweißen erhalten wird.

21. Anordnung nach Anspruch 1, umfassend mindestens zwei des mindestens einen Leitungsabschnitts (38) mit größerem Außendurchmesser als nebenliegenden Abschnitten, welche mindestens zwei Leitungsabschnitte voneinander mit einem axialen Abstand liegen; wobei ein Leitungssegment zwischen den mindestens zwei Leitungsabschnitten angeordnet ist, welches Leitungssegment so ausgebildet ist, um ein U-förmiges gebogenes Segment zu bilden, das mit einer peristaltischen Pumpe (53) operativ verbunden ist.

22. Anordnung nach Anspruch 21, worin das Material zur Herstellung der U-förmigen gebogenen Segmente zur Verwendung mit den peristaltischen Pumpen geeignet ist und vom Material zur Herstellung der beiden Endbereiche der durch der Anschlussbund (39) verbundenen Leitungsabschnitte unterschieden ist.

23. Anordnung nach Anspruch 1, worin der Anschlussbund (39) aus einem steiferen Material besteht als die beiden Endbereiche der durch der Anschlussbund (39) verbundenen Leitungsabschnitte.

24. Montagevorgang für die Anordnung von Elementen nach Anspruch 1, worin der Leitungsabschnitt (38) mit größerem Durchmesser, der zwischen den beiden Anschlägen liegt, in der Befestigungssitz (2) am Tragelement eingeführt wird, wobei die beiden Anschläge dem axialen Paßstift (3a, 3b) in der Befestigungssitz (2) entsprechen, wobei das Tragelement mit einer Abdeckung (8) ausgestattet ist, die dann mit dem Basiskörper (1) verbunden ist, um den Leitungsabschnitt (38) mit größerem Durchmesser mindestens teilweise abzudecken.

25. Integriertes Modul zur extrakorporalen Blutbehandlung, umfassend:
eine Anordnung nach irgendeinem der Ansprüche 1 bis 23;
mindestens eine Blutbehandlungsvorrichtung, die am Tragelement montiert ist und mindestens einen ersten Abteil und mindestens einen zweiten Abteil umfasst, die durch mindestens eine semipermeable Membran voneinander getrennt sind, worin die Gruppe von Fluidverteilungsleitungen mit dem Tragelement verbunden ist und mit der Blutbehandlungsvorrichtung zusammenwirkt.

26. Integriertes Modul nach Anspruch 25, worin die Gruppe von Fluidverteilungsleitungen mindestens eine Leitung nach irgendeinem der Ansprüche 20 bis 23 umfasst.

27. Modul nach irgendeinem der Ansprüche 25 bis 26, worin das Tragelement mindestens einen ersten und mindestens einen zweiten Verbinder (14, 15) umfasst, die mit dem Basiskörper (1) verbunden sind und mit einem Abstand voneinander liegen, zum Empfang und zur Verbindung von entsprechenden Platten einer Blutbehandlungsvorrichtung, die am Tragelement montierbar ist,
wobei die Blutbehandlungsvorrichtung am Basiskörper (1) befestigt ist und durch mindestens ein Paar von Verbindern der ersten, zweiten und dritten Verbinder (14, 15, 16) befestigt ist; und worin
die Blutbehandlungsvorrichtung folgendes umfasst:
- einen Aufnahmekörper;
- mindestens eine semipermeable Membran, die im Aufnahmekörper arbeitet und einen ersten und einen zweiten Abteil definiert;
- einen ersten und einen zweiten Gegenverbinder, die mit dem Aufnahmekörper verbunden sind und an mit dem Basiskörper (1) verbundenen ersten, zweiten und dritten Verbindern (14, 15, 16) befestigt sind, wobei der erste Verbinder (14) und der zweite Verbinder (15) mit dem zweiten Abteil der Blutbehandlungsvorrichtung und mit entsprechenden ersten Endabschnitten (17) des ersten Verbinders (14) und des zweiten Verbinders (15) in Fluidverbindung gesetzt wird;
- mindestens einen Eingangsanschluß zum ersten Abteil und mindestens einen Ausgangsanschluß vom ersten Abteil; und worin
die Gruppe von Fluidverbindungsleitungen mindestens eine Auslaufleitung für gebrauchtes Fluid (35) umfasst, die mit dem zweiten Endabschnitt (18) von einem der ersten, zweiten und dritten Verbinder (14, 15, 16) in Fluidkommunikation gesetzt wird.

28. Modul nach Anspruch 27, worin mindestens eine der Leitungen mit dem Tragelement verbunden ist, wodurch mindestens ein U-förmiges gebogenes Segment am Tragelement definiert ist, das zur Zusammenwirkung beim Betrieb mit einer peristaltischen Pumpe (53) vorgesehen ist.

29. Modul nach Anspruch 28, worin das U-förmige Segment sich außen gegenüber der Umfangswand des Tragelementes erstreckt.

30. Integriertes Modul nach irgendeinem der Ansprüche 25 bis 29, zur Verwendung bei der Behandlung von Niereninsuffizienz, worin die Blutbehandlungsvorrichtung aus einer Gruppe ausgewählt wird umfassend Hämofiltrations-, Hämodialyse-, Hochflussfiltrations- und Hämodiafiltrationsvomichtungen.

## Revendications

1. Série de deux éléments aptes à être couplés l'un à l'autre, où le premier élément est un élément de support pour un module intégré pour le traitement extracorporel de sang et le deuxième élément est un groupe de conduits de distribution pour des fluides, l'élément de support comprenant:
- un corps de base (1);
- au moins une embase de fixation (2) placée sur le corps de base (1) et s'étendant axialement pour loger une portion s'étendant axialement (38) d'un conduit de distribution de fluide;
- au moins un pied de positionnement axial (3a, 3b) rangé dans ladite embase de fixation (2) pour positionner ladite portion axiale de conduit dans une position fixe, ledit au moins un pied de positionnement axial (3a, 3b) étant apte à interagir avec un élément correspondant (39) rangé sur ladite portion axiale (38) de conduit; et
le groupe de conduits de distribution comprenant:
- au moins un conduit ayant au moins une portion (38) de conduit qui présente un diamètre extérieur supérieur à des portions adjacentes, créant ainsi deux butées extérieures; l'au moins une portion (38) à diamètre supérieur comprise entre les deux butées extérieures étant apte à être introduite dans l'embase de fixation (2) rangée sur l'élément de support, **caractérisée en ce que** chaque portion (38) à diamètre extérieur supérieur comprend une collerette de jonction (39) contenant et joignant deux zones d'extrémité de deux parties de conduit relié au fluide.

2. Série selon la revendication 1, où l'élément de support comprend deux pieds de positionnement axiaux (3a, 3b) à une certaine distance axiale mutuelle, entre lesquels la portion axiale de conduit est positionnée dans une position fixe.

3. Série selon la revendication 1 ou 2, où l'embase de fixation (2) comprend un canal ouvert en haut pour l'introduction de la portion axiale de conduit, et où ledit au moins un pied de positionnement axial (3a, 3b) comprend un bord saillant à l'intérieur du canal d'une paroi délimitant le canal.

4. Série selon une quelconque des revendications précédentes, où l'élément de support comprend au moins une paire des embases de fixation (2) rangées l'une à côté de l'autre sur un périmètre du corps de base (1), l'au moins une paire d'embases de fixation recevant deux portions d'extrémité d'un segment en U d'au moins un conduit, le segment en U étant apte à coopérer avec une pompe péristaltique (53).

5. Série selon la revendication 4, où le segment en U saillit à l'extérieur au-delà du périmètre du corps de base (1).

6. Série selon la revendication 4 ou 5, où le corps de base (1) présente une partie plate et en forme de plaque avec une pluralité de côtés périmétriques définissant une forme polygonale du corps de base (1), et où le corps de base (1) présente une pluralité de paires d'embases de fixation (2), chaque paire se composant de deux embases de fixation (2) rangées à côté l'une de l'autre et mutuellement parallèles et rangé sur un de la pluralité de côtés périmétriques.

7. Série selon une quelconque des revendications précédentes, où le pied de positionnement axial (3a, 3b) comprend une surface en contre-dépouille par rapport à une direction axiale de la portion axiale de conduit.

8. Série selon une quelconque des revendications précédentes, où l'embase de positionnement (2) est rangée et configurée pour l'introduction forcée et l'accouplement élastique de la portion axiale de conduit, l'embase de positionnement (2) ayant au moins une ouverture pour l'introduction de la portion axiale de conduit, une section de passage de l'au moins une ouverture étant au moins partiellement inférieure en largeur qu'une largeur maximale de l'embase de fixation (2).

9. Série selon une quelconque des revendications précédentes, où l'embase de fixation (2) est délimitée par une surface apte à recevoir la portion axiale de conduit, laquelle surface présente au moins deux surfaces en contre-dépouille rangées et configurées pour bloquer des déplacements de la portion axiale de conduit par rapport à l'embase de fixation (2) en deux directions de déplacement mutuellement transversales, c'est-à-dire une direction axiale et une direction d'extraction vers le haut à travers d'une ouverture d'introduction supérieure.

10. Série selon une quelconque des revendications précédentes, où l'embase de fixation (2) présente en haut une ouverture pour l'introduction forcée de la portion axiale de conduit, et présente en bas au moins un trou passant (4) en face de l'ouverture d'introduction et à travers lequel il est possible d'exercer une pression d'en bas sur la portion axiale de conduit lorsqu'elle est engagée dans l'embase de fixation (2), permettant ainsi l'extraction de la portion axiale de conduit à travers l'ouverture supérieure pour l'introduction forcée.

11. Série selon une quelconque des revendications précédentes, où:
- l'embase de fixation (2) est définie par un canal ayant une ouverture supérieure pour l'introduction de la portion axiale de conduit;
- le canal s'étend axialement et au moins partiellement saillit axialement dans une direction vers l'extérieur au de-là d'un bord périmétrique du corps de base (1);
- le canal est délimité latéralement par deux parois (5) rangées à côté l'une de l'autre, chacune des deux parois (5) présentant un bord supérieur;
- l'ouverture d'introduction est délimitée par les bords supérieurs des deux parois latérales (5);
- au moins un relief latéral (6) saillit d'au moins une des deux parois latérales (5) et saillit à l'intérieur du canal; l'au moins un relief latéral (6) définissant une surface en contre-dépouille par rapport à une direction d'extraction de la portion axiale de conduit à travers l'ouverture supérieure du canal;
- la surface en contre-dépouille est rangée au-dessous du bord supérieur de la paroi latérale (5).

12. Série selon la revendication 11, où l'élément de support comprend deux reliefs latéraux (6), un pour chaque paroi latérale (5), lesquels deux reliefs latéraux sont en face l'un de l'autre et coopèrent pour bloquer l'extraction de la portion axiale de conduit.

13. Série selon la revendication 11 ou 12, où le canal est apte à relier une portion d'extrémité d'un segment de conduit en U, associable de façon opérationnelle à une pompe péristaltique (53), et où chaque relief latéral (6) se trouve dans une partie d'extrémité du canal, laquelle partie d'extrémité est en face du segment en U.

14. Série selon une quelconque des revendications 11 à 13, où chaque surface en contre-dépouille est inclinée par rapport à une direction d'extraction de la portion axiale de conduit pour permettre l'extraction de la portion axiale de conduit par un mouvement de forcement.

15. Série selon une quelconque des revendications précédentes, où le corps de base (1) a la forme d'une plaque plate et les embases de fixation (2) pour le groupe de conduits de distribution s'étendent sur une face intérieure de ces conduits, et où l'élément de support comprend en outre une couverture (8) pouvant être couplée de façon amovible au corps de base (1) et apte à couvrir au moins une partie de la face intérieure ayant les embases de fixation (2); lorsque la couverture (8) est couplée au corps de base (1), un espace de logement pour au moins une partie du groupe de conduits de distribution est défini entre la face intérieure du corps de base (1) et une face intérieure de la couverture (8).

16. Série selon la revendication 15, où la couverture (8) présente au moins une dent (13) saillant vers le base, qui - lorsque la couverture (8) est couplée au corps de base (1), entre au moins partiellement dans l'embase de fixation (2) et limite un mouvement vers le haut de la portion axiale de conduit reliée dans l'embase de fixation (2).

17. Série selon la revendication 15 ou 16, où la face intérieure du corps de base (1) présente sur un périmètre un bord en élévation (7) pour contenir latéralement au moins une partie du groupe de conduits de distribution, et où la couverture (8) a la forme d'une plaque plate avec un périmètre correspondant au périmètre de la face intérieure du corps de base (1).

18. Série selon la revendication 17, où le corps de base (1) comprend au moins une patte d'accrochage (9) d'une seule pièce avec le corps de base (1) et apte à coupler de façon amovible la couverture (8) au corps de base (1).

19. Série selon une quelconque des revendications précédentes, où l'embase de fixation (2) présente une portion d'extrémité axiale s'étendant axialement à l'intérieur du côté périmétrique du corps de base (1) et une portion d'extrémité s'étendant axialement à l'extérieur du côté périmétrique du corps de base (1).

20. Série selon une des revendications précédentes, où un joint stable de la collerette de jonction (39) avec les deux zones d'extrémité des deux parties de conduit est obtenu par soudage à chaud.

21. Série selon la revendication 1, comprenant au moins deux de l'au moins une portion (38) de conduit ayant un diamètre extérieur supérieur par rapport aux portions adjacentes, lesquelles au moins deux portions de conduits sont à une distance axiale l'une de l'autre, un segment de conduit étant compris entre les au moins deux portions de conduit, lequel segment de conduit est réalisé de sorte à former un segment arqué en U associé de façon opérationnelle à une pompe péristaltique (53).

22. Série selon la revendication 21, où le matériau utilisé pour les segments arqués en U est adapté à l'emploi avec les pompes péristaltiques et est différent du matériau utilisé pour les deux zones d'extrémité des parties de conduit reliées à travers la collerette de jonction (39).

23. Série selon la revendication 1, où la collerette de jonction (39) est en matériau plus rigide que les deux zones d'extrémité des parties de conduits reliées à travers la collerette de jonction (39).

24. Procès d'assemblage de la série d'élément selon la revendication 1, où la portion de conduit (38) ayant un diamètre supérieure et comprise entre les deux butées est introduite dans l'embase de fixation (2) rangée sur l'élément de support, les deux butées correspondant au pied de positionnement axial (3a, 3b) rangé dans l'embase de fixation (2), l'élément de support étant muni d'une couverture (8) qui est ensuite couplée au corps de base (1) pour couvrir au moins partiellement la portion de conduit (38) ayant le diamètre supérieur.

25. Module intégré pour le traitement extracorporel de sang, comprenant:
une série selon une quelconque des revendications 1 à 23;
au moins un dispositif de traitement de sang monté sur l'élément de support, comprenant au moins un premier compartiment et au moins un deuxième compartiment séparés l'un de l'autre par au moins une membrane semi-perméable, où le groupe de conduits de distribution de fluide est associé à l'élément de support et
coopère avec le dispositif de traitement de sang.

26. Module intégré selon la revendication 25, où le groupe de conduits de distribution de fluide comprend au moins un conduit selon une quelconque des revendications 20 à 23.

27. Module selon une quelconque des revendications 25 à 26, où l'élément de support comprend au moins un premier et au moins un deuxième connecteur (14, 15) associés au corps de base (1) et à une certaine distance l'un de l'autre, aptes à recevoir et relier des embases correspondantes d'un dispositif de traitement de sang pouvant être monté sur l'élément de support,
le dispositif de traitement de sang est fixé au corps de base (1) et est fixé par au moins une paire de connecteurs des premiers, deuxièmes et troisièmes connecteurs (14, 15, 16); et où
le dispositif de traitement de sang comprend en outre:
- un corps de logement;
- au moins une membrane semi-perméable opérant à l'intérieur du corps de logement et définissant un premier et un deuxième compartiment;
- un premier et un deuxième contre-connecteur associés au corps de logement et fixés à des premiers, deuxièmes ou troisièmes connecteurs (14, 15, 16) associés au corps de base (1), le premier connecteur (14) et le deuxième connecteur (15) étant mis en liaison de fluide avec le deuxième compartiment du dispositif de traitement de sang et avec des premières portions terminales respectives (17) du premier connecteur (14) et du deuxième connecteur (15);
- au moins une porte d'entrée au premier compartiment et au moins une porte de
sortie du premier compartiment; et où
le groupe de conduits de distribution de fluide comprend au moins un conduit de vidange de fluide usé (35) mis en communication avec la deuxième portion terminale (18) d'une des premiers, deuxièmes et troisièmes connecteurs (14, 15, 16).

28. Module selon la revendication 27, où au moins un des conduits est relié à l'élément de support, définissant au moins un segment arqué en U sur l'élément de support et étant apte à coopérer pendant l'usage avec un pompe péristaltique (53).

29. Module selon la revendication 28, où le segment en U s'étend à l'extérieur par rapport à la paroi périmétrique de l'élément de support.

30. Module intégré selon une quelconque des revendications 25 à 29, pour l'emploi dans le traitement de l'insuffisance rénale, où le dispositif de traitement de sang est choisi d'un groupe comprenant les dispositifs pour hémofiltration, hémodialyse, filtration à flux élevé et hémodiafiltration.
